# EUROPEAN PATENT APPLICATION

(11) **EP 4 672 253 A1**
(43) Date of publication of application: **31.12.2025**
(21) Application number: 24201949.5
(22) Date of filing: 23.09.2024
(51) Int. Cl.: G16H 10/60, G16H 20/10, G16H 20/30, H04L 67/00, G06N 20/00

(54) **ADAPTIVE GENERATION OF PERSONALIZED SCHEDULE FOR DELIVERY OF MESSAGES TO USERS USING MACHINE LEARNING MODELS**

(30) Priority: 27.06.2024 US 202418756322
(71) Applicant: Click Therapeutics, Inc., New York, NY 10013 (US)
(72) Inventor: Morse, William, New York, 10013 (US); Havel, Gunther, New York, 10013 (US); Guttikonda, Sudheer, New York, 10013 (US); Tan, Chenxin, New York, 10013 (US)
(74) Representative: Marks & Clerk LLP

(57) **Abstract**

Aspects of the present disclosure are directed to systems and methods of dynamically generating individualized or personalized times for providing messages over networked environments. The computing system may obtain, for a user device, an event dataset identifying a plurality of interaction times corresponding to a plurality of interactions over an instant time window by a user with an application on the user device to address a condition of the user. The computing system may apply the event dataset to a machine learning (ML) model. The computing system may generate based on applying the event dataset to the ML model, a defined time at which a message is to be provided to the user device during a subsequent time window The computing system may provide for presentation on the user device to address the condition of the user, the message in accordance with the defined time.

## Description

### CROSS REFERENCE TO RELATED APPLICATIONS

The present application claims priority to U.S. Non-Provisional Application No. 18/756,322, titled, "ADAPTIVE GENERATION OF PERSONALIZED SCHEDULE FOR DELIVERY OF MESSAGES TO USERS USING MACHINE LEARNING MODELS", and filed June 27, 2024, and which is incorporated herein by reference in their entireties.

### BACKGROUND

Patients suffering from a medical condition may be directed by a clinician to partake a medical treatment regimen to address the symptoms or the causes of the condition. Poor adherence to such a treatment regimen, however, may be a severe problem in the healthcare field. Patients may find it difficult to stick to the treatment regimen, resulting in no improvement in the condition as well as higher costs on the part of the clinicians from monitoring and on the part of the patients due to additional efforts in trying to adhere to the regimen. There is a need for a method to improve adherence among patients and ultimately influence clinical outcomes. Digital therapeutics may offer some hope at resolving issues related to treatment regimen adherence. In digital therapeutics, messages may be sent and presented to a user's mobile device to deliver as part of evident-based therapeutic interventions directly to the user. The messages may include digital therapeutic content (e.g., in the form of text, audio, or visual content) prompting the user to perform an activity in furtherance of addressing a condition or indication. The user may be directed to perform actions and interact with their device in response to the messages.

One of the technical challenges with digital therapeutics, however, may be in selecting optimal times at which to transmit and present the messages on the user device. This technical challenge may be exacerbated by the consideration that what may constitute optimal times may depend on a number of factors, such as the user, the condition, the type of message being sent, and the particular digital therapeutic intervention, among others. Sending and presenting a message at a sub-optimal time may result in no interactions by the user at the user device. The sub-optimal timing may lead to wasted consumption of computing resources (e.g., processing and memory) on the user device and the server as well as network bandwidth from communicating the message. This may be exacerbated, when application programming interfaces (APIs) provided by another service imposes communication capacity limits. Each time the message is communicated, the API may be invoked, and a portion of the capacity may be spent, resulting in consumption of computing resources. In addition, if the user does not respond at all to the message, the computing resources as a result may be wasted and misallocated. Repeated presentations of messages at sub-optimal times may also result in message blindness on the part of the user, with the user becoming desensitized or unresponsive to messages due to overexposure. Message blindness may thus lead to a degradation of quality of human-computer interactions (HCI) between the user and the user device.

Compounding to this technical challenge, for the therapeutic interventions to be effective at addressing the user's condition, the user should interact with the messages to perform activities as prompted via the user device. Delivery of the messages at sub-optimal times may result in lower or worsened efficacy of the therapeutic intervention on the user. Furthermore, the message blindness may lead to reduced adherence by the user with the digital therapeutic, as exhibited by low or no interaction in response to the messages. This may significantly impact an outcome of the user with the condition, and can prolong symptoms associated with the condition. This may be in addition to the wasted consumption of computing resources and network bandwidth. Approaches at addressing this technical challenge may entail prompting the user to indicate a preference of times at which messages are to be presented, prior to receipt of the messages. These approaches, however, may not be able to readily adapt to the changes as to when the user is most likely to respond to messages and, further, rely on self-reporting or self-awareness of the most optimal times, which may be inaccurate. As a result, such approaches may not resolve the technical issues with sub-optimal times for delivery of messages.

### SUMMARY

Presented herein are systems and methods of adaptively generating a personalized schedule for delivering messages to users using machine learning models, with the objective of maximizing user engagement and managing the user's condition. There are a number of benefits for users of the digital therapeutic applications provided with messages by a message service as disclosed herein. First, by delivering messages to users at optimal times, the performance and effectiveness of the therapeutic solutions in managing the users' conditions are substantially enhanced. This optimal timing reduces the likelihood of message fatigue, ensuring that users remain attentive and engaged when receiving messages on their devices. As a result, users are more inclined to follow through with specified activities and adhere to their treatment regimens. This adherence leads to a marked increase in the overall efficacy of the interventions.

Second, the messaging service leverages machine learning models to dynamically adapt messaging schedules on both population and individual levels, enhancing the user experience and efficacy of interventions. At the population level, the machine learning model categorizes users into distinct groups based on similar behaviors in response to digital therapeutic content. Individual user activity data is then mapped to these groups, allowing for the delivery of messages at optimal times for new users with similar behaviors. This approach ensures that even new users receive messages at times that are most likely to elicit a positive response, based on the behavior patterns of their assigned group. On the individual level, the machine learning model personalizes message delivery schedules by continuously analyzing and adapting to each user's unique behavioral patterns. As more data is collected, the model refines the schedule to optimize message delivery times, ensuring that messages are sent when users are most likely to engage. This personalized approach eliminates biases and inaccuracies that could arise from relying solely on user-indicated preferences, thus providing more effective and timely interventions.

Third, the messaging service collects event data during the onboarding period and throughout the treatment journey to adaptively generate optimal schedules using the machine learning model. This addresses the challenge of limited initial behavioral data towards the beginning of the journey, by leveraging incoming new data to identify the best times to send messages to each user. Even as users' behaviors and preferences change during the course of treatment, the message service is able to continuously update and personalize the schedule of delivery times to remain responsive to the user. By utilizing this data-driven approach paired with machine learning techniques, the messaging service can deliver highly effective and personalized interventions to the users, leading to better health outcomes while shortening of the duration of treatment. The messaging service thus is an improvement over approaches that rely on user-indicated preferences.

Fourth, the messaging streamlines the use of machine learning model, offering significant improvements over other approaches that require constant monitoring of user activity and iterative invocations to identify opportunities to send messages. These other approaches involve an agent or module that persistently tracks user actions to determine the optimal moments to send messages in anticipation of events at the user device. This approach not only demands continuous network connectivity with the user device, but also places a heavy burden on computing resources, especially with computationally complex models that are iteratively called upon to identify opportunities to send messages. This issue may be particularly exacerbated when the API imposes invocation or communication constraints. In contrast, the messaging service may call the timing model that processes batches of interaction data collected over specific time periods in generating schedules. This approach minimizes the frequency of model invocations, effectively reducing computational demands on the service and the consumption of network bandwidth. By limiting the number of times that the model is invoked during the user's journey, the messaging service conserves computing resources and enhances treatment outcomes by delivering timely and relevant communications.

To that end, a messaging service may use a timing model to generate and select an optimal schedule to deliver messages with digital therapeutic content at times at which a user is most likely to interact with the messages to address the user's condition. The messaging service may train a timing model using a training dataset including a set of examples. Each example may include an event dataset. The event dataset may include a set of times corresponding to user interactions with a user device in response to presentation of messages. The messages may have been provided in accordance with a sample schedule. The timing model may be, for example, a clustering model with a feature space with dimensions corresponding to interaction times, among others. The messaging service may generate a feature vector using the interaction times of the event dataset for each example in the training data. With the feature vectors mapped to the dimensions of the feature space, the messaging service may perform clustering analysis to generate a set of regions within the feature space of the timing model. Each region may correspond to a cohort of users with similar behavioral patterns, and may be used to define times at which to deliver messages.

When the user first starts interacting with a digital therapeutic application during on-boarding, the messaging service may receive an initial set of information about the user. For example, the initial information may include a user indicated preference of times. Using the initial information, the messaging service may identify a cohort with a behavioral pattern most similar to the user preference. Based on the identified cohort, the messaging service may select an initiation schedule. The initiation schedule may define a set of times at which a corresponding message (e.g., short-messaging service (SMS), multimedia messaging service (MMS), or in-app messages) is to be delivered to the user device. The set of times may be over a period of time (e.g., 3 days to 1 month).

In accordance with the initiation schedule, the messaging service may send the messages to the user device for presentation. Upon presentation, the user device may monitor for interactions with the digital therapeutic application. Using the interactions aggregated over the time period for the schedule, the user device may generate an event dataset identifying the set of times at which the user interacted with the application upon presentation of messages. The user device may send the event dataset with the aggregated interactions to the messaging service. The messaging service in turn may generate feature vectors to map with the dimensions of the feature space of the timing model. By mapping the feature vectors, the messaging service may identify which region the feature vector falls in. From the identified regions, the messaging service may determine a time (e.g., corresponding to a centroid of the region) for each message to include in a new schedule for the user. The new schedule may define a set of times at which a corresponding message to be delivered to the user device.

In addition, the messaging service may also determine a confidence score for the new schedule. If the confidence score satisfies a threshold, the messaging service may use the new schedule to provide messages to the user device. The new schedule may be particular to the user. Otherwise, if the confidence score does not satisfy the threshold, the messaging service may identify a cohort with a behavioral pattern most similar to the behavioral pattern determined from the event dataset of the user. The messaging service may select another schedule associated with the cohort to use to provide messages to the user device. As more and more event data are gathered from the user device, the messaging service may generate schedules with higher confidence values. The messaging service may also create an instance of the timing model for the user to use in generating schedules personalized to the user.

In this manner, the messaging service may use the timing model to generate schedules to deliver messages at optimal times at which the user is most likely to interact with the digital therapeutic application. By delivering messages at optimal times, the messaging service may reduce and conserve computing resources (e.g., processor and memory) and network bandwidth that would have otherwise been wasted on messages with no responses. The messaging service may also increase efficiency in allocation of such resources by limiting the delivery of messages to times at which the user is most likely to interact. Relative to approaches that rely on continuous communication and invocations of models, the messaging service may reduce the frequency of invocation of the timing model over the course of the digital therapeutic intervention, thereby saving computing resources for other processes. From a user perspective, the provision of messages at optimal times in accordance with the schedule may reduce message blindness, thereby enhancing the quality of human-computer interactions (HCI) between the user and the application. In addition, the messaging service may also improve the adherence by the user with the digital therapeutic provided in the form of messages, thereby improving the user's condition.

Aspects of the present disclosure are directed to systems and methods of generating times for providing messages over networked environments. One or more processors coupled with memory may obtain, for a user device, an event dataset identifying a plurality of interaction times corresponding to a plurality of interactions over an instant time window by a user with an application on the user device to address a condition of the user. The one or more processors may apply the event dataset to a machine learning (ML) model. The ML model may be established using a training dataset including a plurality of examples. Each of the plurality of examples may include a respective event dataset identifying a respective plurality of interaction times corresponding to a respective plurality of interactions over a respective time window by a respective user with a respective application on a respective user device. At least one of the respective plurality of interaction may be detected in response to provision of one or more messages.

The one or more processors may generate based on applying the event dataset to the ML model, a defined time at which a message is to be provided to the user device during a subsequent time window. The one or more processors may provide for presentation on the user device to address the condition of the user, the message in accordance with the defined time.

In some embodiments, the ML model can include a clustering model defining a plurality of clusters established using the plurality of examples. In some embodiments, each cluster of the plurality of clusters corresponding to a respective subset of the plurality of interaction times. In some embodiments, each cluster of the plurality of clusters associated with a respective defined time at which to provide a respective message of the plurality of messages within the set time window. In some embodiments, the one or more processors may compare the plurality of interaction times of the event dataset for the user with one or more of the plurality of clusters of the clustering model. In some embodiments, the one or more processors may identify, from the plurality of clusters, a cluster which corresponds to at least one of the plurality of interaction times. In some embodiments, the one or more processors may use the respective defined time of the cluster as the defined time.

In some embodiments, the one or more processors may compare the plurality of interaction times of the event dataset for the user with one or more of the plurality of clusters of the clustering model. In some embodiments, the one or more processors may identify, from the plurality of clusters, a subset of clusters for a corresponding subset of the plurality of interaction times. In some embodiments, the one or more processors may use the respective defined times of each of the subset of clusters as a plurality of defined times for a schedule.

In some embodiments, the one or more processors may generate a schedule identifying, for each defined time of the plurality of defined times, a respective message type for a corresponding message of the plurality of messages to be provided to the user device. In some embodiments, at least one of the plurality of examples of the training dataset may identify at least one of: (i) a respective schedule identifying a corresponding plurality of defined times at which a respective plurality of messages is to be provided to the respective user device over the set time window, or (ii) a respective indication of whether the respective schedule increased a corresponding response rate of respective plurality of interactions by at least a threshold.

In some embodiments, the one or more processors may generate, based on applying the event dataset to the ML model, a score indicating a degree of confidence for the defined time. In some embodiments, the one or more processors may determine that the defined time is to be used to provide the message to the user device, responsive to the score satisfying a threshold. In some embodiments, the one or more processors may receive a subsequent event dataset identifying a subsequent plurality of interaction times corresponding to a subsequent plurality of interactions over the subsequent time window by the user with the application in response to provision of at least one of the plurality of messages. In some embodiments, the one or more processors may update the ML model using the subsequent event data and the defined time.

In some embodiments, the one or more processors may select, prior to obtaining the event dataset, an initiation schedule identifying an initial plurality of defined times at which an initial plurality of messages is to be provided to the user device over the initial time window, responsive to identifying a lack of prior event datasets for the user. In some embodiments, the one or more processors may provide, for presentation on the user device, the initial plurality of messages in accordance with the initial plurality of defined times of the initiation schedule. In some embodiments, the one or more processors may obtain the event dataset identifying the plurality of interaction times corresponding to the plurality of interactions by the user with the application in response to presentation of at least one of the initial plurality of messages of the initiation schedule.

In some embodiments, the one or more processors may determine from a plurality of categories, a category for the user based on one or more event datasets. In some embodiments, each category of the plurality of categories may associate with a respective behavioral pattern. In some embodiments, the one or more processors may identify an initiation schedule based on the category determined for the user. The initiation schedule may identify an initial plurality of defined times at which an initial plurality of messages is to be provided to the user device. In some embodiments, the event dataset can include at least one of: (i) a health metric associated with the condition of the user, (ii) an interaction rate for the plurality of interactions over the instant time window, or (iii) trait information associated with the user, at least one of the plurality of interactions corresponding to an interaction with the application independent of provision of any message. In some embodiments, the message comprises at least one of a short message service (SMS) message, a multimedia messaging service (MMS), an in-app message, or a chat bot message, wherein the message is to be presented to the user, at least in partial concurrence with the user being on a medication to address the condition.

### BRIEF DESCRIPTION OF THE DRAWINGS

The foregoing and other objects, aspects, features, and advantages of the disclosure will become more apparent and better understood by referring to the following description taken in conjunction with the accompanying drawings, in which:
FIG. 1 depicts a block diagram of a system of adaptive generation of desired times to deliver messages through a digital therapeutics application, in accordance with an illustrative embodiment;
FIG. 2 depicts a block diagram of a process of training a machine learning (ML) model to generate the desired times to deliver messages through the digital therapeutics' application, in accordance with an illustrative embodiment;
FIG. 3 depicts a block diagram of a process of accessing the database to retrieve an initial desired time to generate a schedule to deliver messages through the digital therapeutics application for a user, in accordance with an illustrative embodiment;
FIG. 4 depicts a block diagram of a process of obtaining an event dataset from the digital therapeutics application to generate a new schedule by applying the ML model, in accordance with an illustrative embodiment;
FIG. 5 depicts a block diagram of a process of providing a message according to the new schedule to obtain a subsequent event dataset to update the ML model, in accordance with an illustrative embodiment;
FIG. 6 depicts an example of a visual for the event dataset, in accordance with an illustrative embodiment;
FIG. 7 depicts a flow diagram of a method of adaptive generation of desired times to deliver messages through a digital therapeutics' application, in accordance with an illustrative embodiment; and
FIG. 8 depicts a block diagram of a server system and a client computer system, in accordance with an illustrative embodiment.

### DETAILED DESCRIPTION

For purposes of reading the description of the various embodiments below, the following enumeration of the sections of the specification and their respective contents may be helpful:

Section A describes systems and methods for adaptive generation of schedules for delivery of messages to address conditions in users; and

Section B describes a network and computing environment which may be useful for practicing embodiments described herein.

### A. Systems and Methods for Adaptive Generation of Schedules for Delivery of Messages to Address Conditions in Users

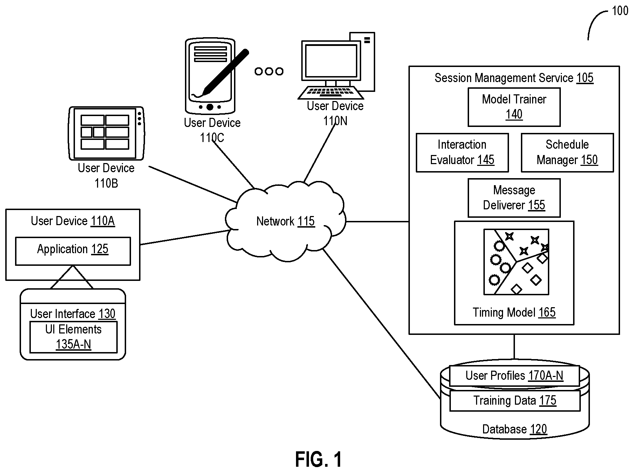

Referring now to FIG. 1, depicted is a block diagram of a system 100 of adaptive generation of schedules to deliver messages to address conditions. In an overview, the system 100 may include at least one session management service 105, a set of user devices 110A-N (hereinafter generally referred to as user devices 110, and a database 120, communicatively coupled with one another via at least one network 115. At least one of the user devices 110 (e.g., the first user device 110A as depicted) may include at least one application 125. The application 125 may include or provide at least one user interface 130 with one or more user interface (UI) elements 135A-N (hereinafter generally referred to as UI elements 135). The session management service 105 may include at least one model trainer 140, at least one interaction evaluator 145, at least one schedule manager 150, at least one message deliverer 155, and at least one timing model 165, among others. The session management service 105 may include or have access to at least one database 120. The functionalities of the application 125 on the user device 110 may be performed in part on the session management service 105, and vice versa. Each of the components of the system 100 can be implemented using the computing system as described in Section B.

In further detail, the session management service 105 (sometimes herein generally referred to as a messaging service, service, or server) may be any computing device comprising of one or more processors coupled with memory and software and capable of performing the various processes and tasks described herein. The session management service 105 may be in communication with the one or more user devices 110 and the database 120 via the network 115. The session management service 105 may be situated, located, or otherwise associated with at least one computer system. The computer system may correspond to a data center, a branch office, or a site at which one or more computers corresponding to the session management service 105 are situated.

On the session management service 105, the model trainer 140 may train, improve, or update the timing model 165 related to a message provided by the session management service for a user of the application 125. The interaction evaluator 145 may evaluate the interactions within the event dataset and transmit indications to the schedule manager 150. The schedule manager 150 may extract and transmit schedules to and from the various components of the system 100. The message deliverer 155 may transmit messages from the schedule to the user devices 110. The timing model 165 may be any machine learning model for estimating or predicting a time and day during which the user interacts with the application 125 on the user devices 110. The timing model 165 may be any clustering algorithm or model used to perform clustering analysis on response data associated with the user in performing the activities. The clustering model may include, for example, hierarchical clustering (e.g., linkage clustering), centroid-based clustering (e.g., *k*-means), distribution-based clustering (e.g., Gaussian mixture models), and density-based clustering (e.g., density-based spatial clustering or DBSCAN), among others. The timing model 165 may be particular to a given user or a cohort of users.

The user device 110 (sometimes herein referred to as an end user computing device) may be any computing device comprising one or more processors coupled with memory and software and capable of performing the various processes and tasks described herein. The user device 110 may be in communication with the session management service 105 and the database 120 via the network 115. The user device 110 may be a smartphone, other mobile phone, tablet computer, wearable computing device (e.g., smart watch, eyeglasses), or laptop computer. The user device 110 may be used to access the application 125. In some embodiments, the application 125 may be downloaded and installed on the user device 110 (e.g., via a digital distribution platform). In some embodiments, the application 125 may be a web application with resources accessible via the network 115.

The application 125 executing on the user device 110 may be a digital therapeutics application. The application 125 may present or provide a session (sometimes referred to herein as a therapy session) to address at least one condition in the user. The condition of the end user may include, for example, chronic pain (e.g., associated with or including arthritis, migraine, fibromyalgia, back pain, Lyme disease, endometriosis, repetitive stress injuries, irritable bowel syndrome, inflammatory bowel disease, and cancer pain), a skin pathology (e.g., atopic dermatitis, psoriasis, dermatillomania, and eczema), a cognitive impairment (e.g., mild cognitive impairment (MCI), Alzheimer's, multiple sclerosis, and schizophrenia), a mental condition (e.g., an affective disorder, depression, bipolar disorder, obsessive-compulsive disorder, borderline personality disorder, and attention deficit/hyperactivity disorder), a substance use disorder (e.g., opioid use disorder, alcohol use disorder, tobacco use disorder, or hallucinogen disorder), and other conditions (e.g., narcolepsy and oncology or cancer), among others.

The end user may be taking or being administered with a medication to address the condition, in at least partial concurrence with the use of the application 125 (e.g., for any number of sessions). For instance, if the medication is for pain, the end user may be taking acetaminophen, a nonsteroidal anti-inflammatory composition, an antidepressant, an anticonvulsant, or other composition, among others. For skin pathologies, the end user may be taking a steroid, antihistamine, or topic antiseptic, among others. For cognitive impairments, the end user may be taking cholinesterase inhibitors or memantine, among others. For a mental condition, the end user may be taking antidepressants, mood stabilizers, antipsychotics, anxiolytics, or stimulants, among others. For substance abuse disorders, the end user may be taking a naltrexone, disulfiram, acamprosate, or nicotine replacement therapy, among others. The application 125 may increase efficacy of the medication that the user is taking to address the condition.

The end user may also participate in other psychotherapies for these conditions. In some embodiments, the digital therapeutic content may be provided to the end user within the digital therapeutics application towards achieving an endpoint of the end user. An endpoint can be, for example, a physical or behavioral goal of an end user, a completion of a medication regimen, or an endpoint indicated by a doctor or an end user. At least one of the end user devices 110 may have a digital therapeutics application and may provide a session (sometimes referred to herein as a therapy session) to address at least one condition of the end user.

The database 120 may store and maintain various resources and data associated with the session management service 105 and the application 125. The database 120 may include a database management system (DBMS) to arrange and organize the data maintained thereon, as the user profiles 170, and the training data 175, among others. The database 120 may be in communication with the session management service 105 and the one or more user devices 110 via the network 115. While running various operations, the session management service 105 and the application 125 may access the database 120 to retrieve identified data therefrom. The session management service 105 and the application 125 may also write data onto the database 120 from running such operations.

On the database 120, each user profile 170 (sometimes herein referred to as a user account, user information, or subject profile) can store and maintain information related to a user of the application 125 through user device 110. Each user profile 170 may be associated with or correspond to a respective user of the application 125. The user profile 170 may identify various information about the user, such as a user identifier, the condition to be addressed, information on sessions conducted by the user (e.g., activities or lessons completed), message preferences, user trait information, and a state of progress (e.g., completion of endpoints) in addressing the condition, among others. The information on a session may include various parameters of previous sessions performed by the user and may be initially null. The message preferences can include treatment preferences and user input preferences, such as types of messages or timing of messages preferred. The message preferences can also include preferences determined by the session management service 105, such as a type of message the user may respond to. The progress may initially be set to a start value (e.g., null or "0") and may correspond to alleviation, relief, or treatment of the condition. The user profile 170 may be continuously updated by the application 125 and the session management service 105.

In some embodiments, the user profile 170 may identify or include information on a treatment regimen undertaken by the user, such as a type of treatment (e.g., therapy, pharmaceutical, or psychotherapy), duration (e.g., days, weeks, or years), and frequency (e.g., daily, weekly, quarterly, annually), among others. The user profile 170 can include at least one activity log of messages provided to the user, interactions by the user identifying performance of the specific user, and responses from the user device 110 associated with the user, among others. The user profile 170 may be stored and maintained in the database 120 using one or more files (e.g., extensible markup language (XML), comma-separated values (CSV) delimited text files, or a structured query language (SQL) file).

The database 120 may store and maintain the set of messages for addressing the condition of the user of the application 125. Each message may include digital therapeutic content. The messages may be transmitted, sent, or otherwise provided by the session management service 105 to the user device 110 for presentation. The messages itself may be in any format. In some embodiments, at least one message may be a short message/messaging service (SMS) message. The SMS message may include a text of a set length (e.g., limit to number of alphanumeric characters) to be transmitted over cellular networks. In some embodiments, at least one message may be a multimedia messaging service (MMS) message. The MMS message may include multimedia content, such as text, images, video, or audio, among others. In some embodiments, at least one message may be an in-app message. The in-app message may include instructions to present multimedia content (e.g., in the form of text, images, video, or audio content or any combination thereof) via UI elements of the user interface of the application 125. The instruction may identify or specify one or more UI elements to be rendered, displayed, or otherwise presented within the user interface of the application 125.

The digital therapeutic content of the message may be in any modality, such as text, image, audio, video, or multimedia content, among others, or any combination thereof. The messages can be stored and maintained in the database 120 using one or more files. For instance, for text, the digital therapeutic content can be stored as text files (TXT), rich text files (RTF), extensible markup language (XML), and hypertext markup language (HTML), among others. For an image, the digital therapeutic content may be stored as a joint photographic experts' group (JPEG) format, a portable network graphics (PNG) format, a graphics interchange format (GIF), or scalable vector graphics (SVG) format, among others. For audio, the digital therapeutic content can be stored as a waveform audio file (WAV), motion pictures expert group formats (e.g., MP3 and MP4), and Ogg Vorbis (OGG) format, among others. For video, the digital therapeutic content can be stored as a motion pictures expert group formats (e.g., MP3 and MP4), QuickTime movie (MOV), and Windows Movie Video (WMV), among others. For multimedia content, the digital therapeutic content can be an audio video interleave (AVI), motion pictures expert group formats (e.g., MP3 and MP4), QuickTime movie (MOV), and Windows Movie Video (WMV), among others.

In some embodiments, the digital therapeutic content of the message may include a set of stimuli (e.g., in the form of audio, visual, or text) for the user to conduct a particular task. The task may include, for example, an implicit association task (IAT) (e.g., associating stimuli with concepts); an attention bias modification training (ABMT) (e.g., training users to shift attention away from certain stimuli); an emotional faces memory task (EFMT) (e.g., testing users to recognize and remember certain facial emotions); digital support tool (DST) (e.g., providing messages based on state of user); and adaptive goal setting (AGS) (e.g., providing messages based on dynamic objectives for user); among others. Although the messages are described herein in terms of digital therapeutic content, the messages can include other types of content.

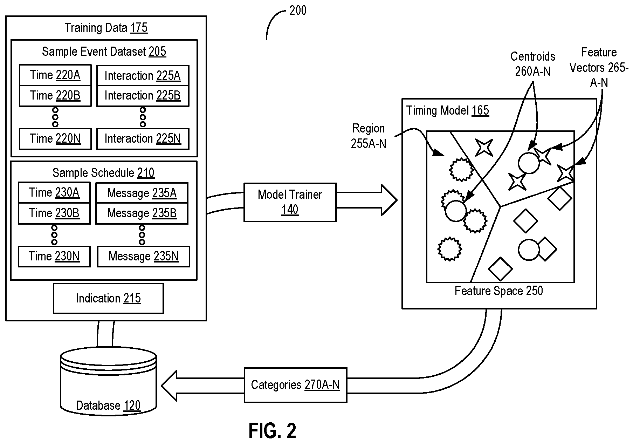

Referring now to FIG. 2, depicted is a block diagram of a process 200 of training the timing model 165 to generate the desired times to deliver messages through the digital therapeutics' application. The process 200 may include or correspond to operations performed by the system 100 to train the timing model 165. Under the process 200, the model trainer 140 my train, initiate, or otherwise establish the timing model 165. The model trainer 140 may train the timing model 165 to be in accordance with the architecture of the timing model 165. For example, when the timing model 165 is a cluster model, the training may be iteratively performed until convergence of the clusters, adequacy of clustering results, convergence of the algorithm, or completion of post-training validation in accordance with learning for the clustering model. The training data 175 may access, retrieve or otherwise obtain training data 175 from the database 120.

The training data 175 may include a set of examples used to train the timing model 165. The set of examples may be constructed or generated using historical data of interactions and message delivery schedules. For each example, the training data 175 may include at least one sample event dataset 205. The sample event dataset 205 may include a set of times 220A-N (generally referred to as interaction times 220) and set of interactions 225A-N (generally referred to as interactions 225). Each interaction time 220 may identify a time of day, a day of week, or a date (e.g., year, month, and day), among others for the associated interaction 225. In some embodiments, the interaction time 220 may be relative to another set reference time, such as a start of therapy (e.g., initial use of the application 125) or a start of week (e.g., Sunday or Monday), among others. The set of times 220 may be over a time window ranging between 3 hours to 3 months. The interaction 225 may identify a type of interaction (e.g., mouse click, a swipe, a hover over, tilt, or a screen touch), a type of activity (e.g., performed by the user 302 and recorded via the application 125), an indication of whether the activity was performed, among others.

The sample event dataset 205 can include a health metric associated with the condition of the user. The health metric may indicate a degree of health for the current user with respect to the condition (e.g., symptom or severity). For instance, a user who has not made progress within the application 125 may have a lower health metric. The sample event dataset 205 may have an interaction rate for the set of interactions 225. The interaction rate may identify a frequency of interactions 225 over a duration of the set of times 220. The sample event dataset 205 may include trait information associated with the user. The trait information may identify or include, for example, age, location, or device type, among others. In some embodiments, at least one of the set of interactions 225 may correspond to an interaction with the application 125, independent of provision of any message. For instance, the sample event dataset 205 may include interactions 225 by users corresponding to logging into the application 125.

In some embodiments, the training data 175 may include at least one sample schedule 210. The sample schedule 210 may identify a set of delivery times 230A-N (generally referred as delivery times 230) and a set of messages 235A-N (generally referred to as messages 235). Each message 235 may have been previously transmitted at a corresponding delivery time 230. Each delivery time 230 may identify a time of day, a day of week, or a date (e.g., year, month, and day), among others for the associated message 235. The set of delivery times 230 may be over a time window ranging between 6 hours to 3 months. The sample schedule 210 may be associated with the sample event dataset 205. For instance, at least some of the interactions 225 of the sample event dataset 205 may be in response to the presentation of messages 235. For each delivery time 230, the sample schedule 210 may identify a type of message or a message identifier (e.g., referring to a specific message), among others. The message 235 may include at least one of a short message service (SMS) message, a multimedia messaging service (MMS), an in-app message, or a chat bot message.

In some embodiments, the training data 175 may include an indication 215. The indication 215 may be associated with the sample event dataset 205 and the sample schedule 210. The indication 215 may identify whether the sample schedule 210 resulted in an increased (or decreased) interaction rate in the set of interactions 225 of the sample event dataset 205. The increased interaction rate may be at least by a threshold. In some embodiments, the model trainer 140 may select the example (including the sample event dataset 205 and the sample schedule 210) from the training data 175 based on the indication 215. When the increase in interaction rate satisfies (e.g., greater than or equal to) the threshold, the model trainer 140 may select the example to use in training the timing model 165. Otherwise, when the increase in interaction rate does not satisfy (e.g., less than) the threshold, the model trainer 140 may exclude the example to use in training the timing model 165.

With the training data 175, the model trainer 140 may initiate, train, and establish the timing model 165. The timing model 165 may include at least one feature space 250. The feature space 250 may be an n-dimensional space in which each feature vectors 265A-N (hereinafter generally referred to feature vector 265) can be defined, referenced, or mapped. The dimensions of the feature space 250 may include, for example, interaction 225 or interaction time 220, among others. Each feature vector 265 may be a data point corresponding to at least one interaction time 220 at which the user interacts with the application 125. In some embodiments, each feature vector 265 may be a data point corresponding to the interaction time 220, the interaction 225, the delivery time 230, and the message 235, or any combination thereof, among others. For instance, the dimensions of the feature vector 265 may correspond to the delivery time 230 at which a given message 235 is provided, the interaction 225 in response to presentation of the message 235, and the interaction time 220. The dimension associated with time may encompass the range of values for the time window (e.g., 6 hours to 3 months) associated with the sample event dataset 205 or the sample schedule 210. The feature vector 265 may be defined or may correspond to a set of coordinates within the n-dimensions of the feature space 250. The feature space 250 may define or otherwise include a set of clusters or centroids 260A-N (hereinafter generally referred to as centroids 260). Each centroid 260 may correspond to a point in the n-dimensional feature space 250.

Continuing on, the set of centroids 260 may be used to delineate, demarcate, or otherwise define a corresponding set of regions 255A-N (hereinafter generally referred to as regions 255, and sometimes referred to herein as clusters) within the feature space 250. The number of centroids 260 and the number of regions 255 may be pre-set or pre-assigned to a value (e.g., a fixed value). For example, the number of centroids 260 and regions 255 may correspond to the number of different categories of users. Each region 255 may correspond to a portion of the feature space 250. Each region 255 may be associated with or may be used to determine at least one delivery time at which to provide a respective message over the time window In some embodiments, each region 255 may correspond to the portion of the feature space 250 based on a distance about the associated centroid 260 in the feature space 250. The distance may be, for example, proximity in terms of Euclidean distance or L-norm distance, among others, to the centroid 260 defining the respective region 255.

In establishing the timing model 165, the model trainer 140 may parse the sample event dataset 205 to identify the set of interactions 225 associated with the interaction time 220. For each interaction 225, the model trainer 140 may generate a data point as a feature vector 265 to include within the feature space 250 of the timing model 165. The model trainer 140 may convert the interaction time 220 to the data point of the feature vector 265. The feature vector 265 may include a set of coordinates in the feature space 250 corresponding to the interaction time 220. In some embodiments, the model trainer 140 may convert the interaction time 220, the interaction 225, the delivery time 230, and the message 235 to a corresponding feature vector 265. The feature vector 265 may include a set of coordinates in the feature space 250 corresponding to the interaction time 220, the interaction 225, the delivery time 230, and the message 235, among others. The model trainer 140 may generate the feature vector 265 and map the feature vector 265 within region 255 of the feature space 250. In training the timing model 165, the model trainer 140 may repeat the generation of the feature vector 265 for each interaction 225 of the sample event dataset 205.

Using the feature vectors 265, the model trainer 140 may identify, generate, or otherwise determine the set of centroids 260 and the set of regions 255 in the feature space 250 of the timing model 165. Since the interactions 225 may associate with an interaction time 220 of the day, the model trainer 140 may assign the at least one centroid 260 corresponding to the interaction time 220 corresponding to the coordinates of the one feature vector 265. The model trainer 140 may define the region 255 based on the distance about the centroid 260, such as a proximity in terms of Euclidean distance or L-norm distance. From here, the model trainer 140 may maintain the timing model 165 by using a collection of interactions 225 and the interaction time 220 (i.e., past and current) associated with the interactions 225 to fine tune and adjust the timing model 165. Once trained, the model trainer 140 may store, generate, or identify an association between the timing model 165.

With the creation of the regions 255, the centroids 260, and the feature vectors 265 within the feature space 250, the timing model 165 may generate, determine, or otherwise create new centroids 260 within the respective region 255. The new centroids 260 may correspond to the interactions 225 and associate with defined delivery times 230 to provide the messages 235 using the association between the user and the timing model 165. In this manner, the timing model 165 may produce a defined time at which to provide the message for the respective user. In some embodiments, the timing model 165 may generate, create or otherwise generate a schedule for the user identifying the messages to send within the defined window based on the region 255, centroid 260, and the feature vectors 265. From here, the model trainer 140 may compare the generated defined time with the delivery times 230 within the sample schedule 210. The model trainer 140 may identify a level of deviation based on the comparison. The model trainer 140 may use the indication 215 in accordance with the level of deviation to identify whether the generated defined times increased a corresponding response rate of the messages 235. Using the indication 215, the model trainer 140 may adjust the regions 255, centroids 260, and the feature vectors 265 to create defined time with a positive indication 215, and repeat the process 200 until the timing model 165 is trained beyond a threshold or with positive indication 215 for each user.

In some embodiments, the model trainer 140 may determine, create, or otherwise generate a set of categories 270A-N using the feature space 250 of the timing model 165. Each category 270 may correspond to at least one region 255 (or cluster). Each category 270 may correspond to a group (or cohort) of users with similar behavioral patterns based on interaction times 220 used to generate the feature vector 265. Each group may include one or more users with interactions 225 at similar interaction times 220. For instance, a first region 255Amay correspond with a category 270A in which users interact in the morning upon presentation of messages 235. A second region 255B may correspond with another category 270B in which users interact in the evening upon presentation of messages 235.

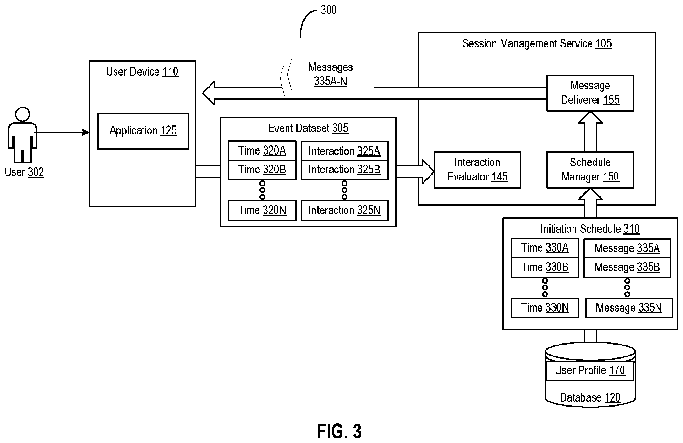

Referring now to FIG. 3, depicted is a block diagram of a process 300 of accessing the database 106 to retrieve an interaction time 320 to generate a schedule 304 to deliver messages 306 through the digital therapeutics application for a user 302. The process 300 may include or correspond to operations performed by the system 100 to provide messages using an initiation schedule. The session management service 105 may generate, create, or otherwise establish at least one user profile 170 for each user 302 of the application 125. The application 125 may present the user with a plurality of questions, prompts, images, videos, among others to generate the user profile 170. For instance, the user 302 may specify a name, a date of birth, an initial time to receive messages for the application 125, conditions of the user 302, medications for the user 302, among others. In some instances, a physician may provide information associated with the user 302 to the respective user profile 170 of the user 302. From here, the application 125 may transmit the information of the user 302 to the session management service 105 to generate the respective user profile 170.

Using the user profile 170, the schedule manager 150 may identify or select at least one initiation schedule 310 for the user 302. The initiation schedule 310 may be selected in response to any event data for the user 302. The initiation schedule 310 may be used during an on-boarding stage for the user 302 with respect to the application 125. The initiation schedule 310 can include a set of delivery times 330A-N (generally referred to as delivery times 330) and a corresponding set of messages 335A-N (generally referred to as messages 335). The initiation schedule 310 may indicate the delivery time 330 at which to transmit at least one message 335. Each delivery time 330 may identify a time of day, a day of week, or a date (e.g., year, month, and day), among others for the associated message 335. The set of delivery times 330 may be over a time window ranging between 6 hours to 3 months. For each delivery time 330, the initiation schedule 310 may identify a type of message or a message identifier (e.g., referring to a specific message), among others. The message 335 may include at least one of a short message service (SMS) message, a multimedia messaging service (MMS), an in-app message, or a chat bot message. The message 335 may be presented to the user 302 via the application 125 or directly through the user device 110. For instance, the message 335 may appear as a notification within the application 125. In some embodiments, the message 335 may be provided in partial concurrence with the user 302 being on a medication to address the condition.

In some embodiments, the schedule manager 150 may access the database 120 to extract or identify a set of initiation schedules 310. Using the user profile 170, the schedule manager 150 may identify, or otherwise select, the initiation schedule 310 associated with a user profile type corresponding to the user profile 170 of the user 302. Each of the initiation schedules 310 may be associated with a user profile type. In operation, the schedule manager 150 may receive or retrieve information about the user (e.g., medications, conditions, free time, work hours, etc.) from the user profile 170. For instance, the schedule manager 150 may use the work hours of the user 302 to select the initiation schedule 310 with the corresponding profile type. In another instance, the schedule manager 150 may use the medications, the conditions, and the age of the user 302 to select the initiation schedule 310.

In some embodiments, the schedule manager 150 may select the initiation schedule 310 based on the application history. For instance, the user device 110 may include previous versions of the application 125 through the duration of the user 302 interacting with the application 125. The application 125 may track, monitor, or otherwise detect an interaction time 320 for the user 302 and store the interactions within a log of interactions within the memory of the user device 110. In some embodiments, the schedule manager 150 may select the initial schedule based on the population group. The interaction evaluator 145 may use the application history and geographic location to determine habits associated with each user 302 of a similar geographic location within the population group.

The message deliverer 155 may output, transmit, or otherwise provide at least one message 335 at the delivery time 330 for presentation on the user device 110. When the schedule manager 150 extracts the delivery time 330 to send the one or more messages 335, the message deliverer 155 may create, generate, or use a data structure to store the messages 335 until the associated delivery time 330 occurs. The message deliverer 155 may store the messages 335 in, for example, a queue, a stack, a linked list, an array, a hash map, a heap, a multiset, among others, until the delivery time 330 occurs. For instance, the message deliverer 155 may store the messages 335 in a linked list where the head of the linked list is the first message 33 5A in the early morning delivery time 330A and the tail of the linked list is the last message 335B in the late night.

The message deliverer 155 may transmit, send, or otherwise provide the message to the user device 110, responsive to the delivery time 330 for the message 335 occurring. The message deliverer 155 may transmit multiple messages 335 to the user device 110 in accordance with the set of delivery times 330. In some embodiments, the message deliverer 155 may transmit the plurality of messages 335 at different delivery times 330 throughout the day in accordance with the set of times. Each message 335 may correspond to at least one delivery time 330 within the schedule. For instance, according to the initiation schedule 310, the message deliverer 155 may transmit a first message 335A, a second message 335B, and a third message 335C at a delivery time 330Ain the early morning to the user device 110. However, the message deliverer 155 may transmit a fourth message 335D and a fifth message 335E at a delivery time 330B in the late afternoon to the user device 110.

Upon receipt, the user device 110 may present, provide, or otherwise display the message 335. In some embodiments, the application 125 on the user device 110 may present the message 335 using the UI elements of the user device 110. The messages 335 may correspond to treatments, therapeutics, exercises, therapy, and medications, among others, for the user 302 to do or execute. For instance, the message 335 may encourage the user 302 to conduct breathing exercises to treat asthma. In another instance, the messages 335 may encourage the user 302 to ingest tablets for pain associated with lung cancer. In another instance, the message 335 may encourage the user 302 to perform physical therapy exercises for leg surgery recovery. By presenting the messages 335 at the delivery time 330, the user device 110 may capture more interactions between the user 302 and the application 125. The user device 110 (or the application 125) may monitor for interactions by the user 302 with the user device 110, in response to the provision of the message 335.

As the user device 110 detects, captures, and otherwise identifies interactions, the user device 110 may generate at least one event dataset 305. In some embodiments, the application 125 may generate the event dataset 305 for an interval (e.g., every 1 day, every 5 days, or every 2 weeks) within the time window (e.g., 6 hours to 3 months) for the initiation schedule 310. In some embodiments, the application 125 may generate the event dataset 305 at or towards (e.g., within 1 to 3 days) an end of the time window for the initiation schedule 310. The event dataset 305 may include a set of interaction times 320A-N (hereinafter generally referred to as interaction times 320) and a corresponding set of interaction 325A-N (hereinafter generally referred to as interactions 325). Each interaction time 320 may identify a time of day, a day of week, or a date (e.g., year, month, and day), among others for the associated interaction 325. In some embodiments, the interaction time 320 may be relative to another set reference time, such as a start of therapy or a start of week, among others.

Continuing on, the interaction 325 may identify a type of interaction (e.g., mouse click, a swipe, a hover over, tilt, or a screen touch), a type of activity (e.g., performed by the user 302 and recorded via the application 125), an indication of whether the activity was performed, among others. The user device 110 may generate the event dataset 305 by using the application 125 to collect, store, or otherwise capture each interaction 325 and the respective interaction time 320. Each interaction time 320 may be a length of time or an instant of time. For instance, an interaction time 320A may correspond to a plurality of interactions 325 occurring within a time window of 2-6 minutes. In another instance, an interaction time 320A may correspond to a single interaction 325A.

The user device 110 may transmit, send, or otherwise provide the event dataset 305 to the interaction evaluator 145. The user device 110 may transmit a sending request to the session management service 105. The sending request may indicate that the user device 110 is online and has an event dataset 305 to send. The sending request may include metadata associated with the event dataset 305 such as the size of the event dataset 305, the duration in which the user device 110 was offline, the user device 110 corresponding to the event dataset 305, the device ID, among others. Upon connecting, the user device 110 may transmit the event dataset 305 to the session management service 105.

The interaction evaluator 145 may obtain, receive, or otherwise retrieve the event dataset 305 from the user device 110. Upon receipt, the interaction evaluator 145 may parse the event dataset 305 to extract or identify the set of interaction times 320 and the corresponding set of interactions 325. To evaluate the initiation schedule 310, the interaction evaluator 145 may compare the delivery time 330 with the initiation schedule 310 with the interaction time 320 within the event dataset 305 and output a performance score. The performance score may indicate a level of effectiveness for the set of delivery times 330 of the initiation schedule 310 in accordance with the number of interactions 325. In some instances, the interaction time 320 within the event dataset 305 may not deviate from the time within the initiation schedule 310 indicating a performance score of high effectiveness of the initiation schedule 310. In some instances, the interaction time 320 within the event dataset 305 may deviate from the time within the initiation schedule 310 indicating a performance score of low effectiveness of the initiation schedule 310.

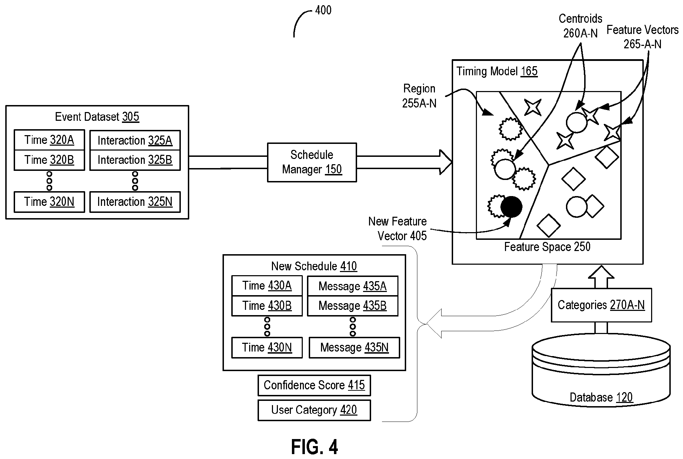

Referring now to FIG. 4, depicted is a block diagram of a process 400 of obtaining an event dataset 305 from the application 125 to generate a new schedule 410 by applying the timing model 165. The process 400 may include or correspond to operations performed by the system 100 to generate the new schedule 410 by applying the timing model 165 to the event dataset 305. Under the process 400, the schedule manager 150 may receive, retrieve, or otherwise obtain the event dataset 305 from the interaction evaluator 145. Upon receiving the event dataset 305, the schedule manager 150 may extract the interactions 325, interaction times 320. The schedule manager 150 may also use performance score from the interaction evaluator 145 to determine whether to update the timing model 165.

The schedule manager 150 may feed, apply, or provide the event dataset 305 to the timing model 165 to generate at least one new schedule 410 (sometimes herein referred to as a subsequent schedule). The new schedule 410 may be similar in structure as the initiation schedule 310. The new schedule 410 can include a set of new delivery times 430A-N (generally referred to as new delivery times 430) and a set of respective messages 435A-N (generally referred to as messages 435) for presentation to the user 302. Each delivery time 430 may identify a time of day, a day of week, or a date (e.g., year, month, and day), among others for the associated message 435. The set of delivery times 430 may be over a time window ranging between 3 days to 1 month. The time window for the new schedule 410 may be subsequent to the time window for the initiation schedule 310. For each delivery time 430, the new schedule 410 may identify a type of message or a message identifier (e.g., referring to a specific message), among others. The message 435 may include at least one of a short message service (SMS) message, a multimedia messaging service (MMS), an in-app message, or a chat bot message.

In feeding, the schedule manager 150 may calculate, determine, or otherwise generate at least one feature vector 405. In some embodiments, the schedule manager 150 may generate the feature vector 405 for each interaction time 320 in the sample event dataset 205. In some embodiments, the schedule manager 150 may generate the feature vector 405 for a combination of the interaction time 320, the interaction 325, the delivery time 330, and message 335, among others. For instance, the feature vector 405 may represent a data point generated as a function of the interaction time 320, the interaction 325 (e.g., type of interaction), and the delivery time 330 for a given message 335.

With the generation of the new feature vector 405, the schedule manager 150 may map or compare the new feature vector 405 against the feature space 250 of the timing model 165. By comparing the feature vector 405 for each interaction time 320, the schedule manager 150 may compare the set of interaction time 320 with the regions 255 of the feature space 250. For each new feature vector 405, the schedule manager 150 may select or identify the region 255 in the feature space 250 corresponding to the interaction time 320. The schedule manager 150 may use the region 255 to identify a corresponding delivery time 430 to include in the new schedule 410. With the identification, the schedule manager 150 may determine or identify the centroid 260 defining the region 255. Based on the centroid 260, the schedule manager 150 may calculate or determine a corresponding delivery time 430 to insert the new schedule 410. In some embodiments, using the centroid 260, the schedule manager 150 may also select or identify the corresponding message 435 for the delivery time 430. The schedule manager 150 may repeat this process over all the new feature vectors 405 corresponding to the interaction times 320 of the sample event dataset 205 to produce the new schedule 410. The new delivery times 430 may indicate when the messages 435 are to be presented based on the behavioral pattern of the user 302. For instance, a user 302 may have a change in work hours and interact with the application 125 earlier than the initial delivery time 330. Using the earlier interaction, the timing model 165 may generate a new delivery time 430 to adapt to the change in behavioral pattern for the particular user 302.

Concurrently to generating the new schedule 410, the schedule manager 150 may use the timing model 165 to generate, create, or otherwise calculate at least one confidence score 415 for the new schedule 410. The confidence score 415 may indicate a degree of confidence that the set of delivery times 430 in the new schedule 410 is optimal for the user 302. For instance, the confidence score 415 may indicate the degree of confidence that the user 302 will likely respond in response to provision of the messages 435 at the set of delivery times 430 specified by the new schedule 410. The confidence score 415 may be determined as a function of the new feature vector 405 for each interaction time 320 and the feature vectors 265 in the feature space 250 of the timing model 165. In some embodiments, the schedule manager 150 may determine the confidence score 415 based on a distance between the new feature vector 405 and the centroid 260 in the region 255. In some embodiments, the schedule manager 150 may calculate a silhouette coefficient as the confidence score 415 based on the new feature vector 405 and the feature vectors 265 within the same region 255. The silhouette coefficient may indicate a degree of similarity of feature vectors 265 and the feature vector 405 within the region 255. The schedule manager 150 may repeat the determination over all the feature vectors 405, and generate an aggregate value (e.g., a weighted sum or average) for the confidence score 415.

To determine whether to use the new schedule 410, the schedule manager 150 may compare the confidence score 415 with a threshold. The threshold may identify, define, or delineate a value for the confidence score 415 at which to select the new schedule 410 for use in delivering messages 435 to the user 302. When the confidence score 415 satisfies (e.g., greater or equal than) the threshold, the schedule manager 150 may determine, identify, or otherwise indicate that the new schedule 410 may be used. Otherwise, when confidence score 415 does not satisfy (e.g., less than) the threshold, the schedule manager 150 may determine, identify, or otherwise indicate that the new schedule 410 may not be used.

In some embodiments, the schedule manager 150 may use the timing model 165 to identify or select at least one category 270 for the user 302. When the confidence score 415 does not satisfy the threshold, the schedule manager 150 may use the timing model 165 to select or identify the category 270 for the user 302. To identify, the schedule manager 150 may identify or determine a behavioral pattern associated with the user 302. In some embodiments, the schedule manager 150 may determine a behavioral pattern using the event dataset 305. The behavioral pattern may characterize interactions 325 performed by the user 302 with the application 125. For example, if the interactions 325 indicate that the user 302 interacts more frequently with the application 125 in the morning, the schedule manager 150 may classify the behavioral pattern of the user 302 as a morning interactor.

In some embodiments, the schedule manager 150 may determine the behavioral pattern from the new feature vectors 405 generated from the interaction times 320 of the interactions 325. The schedule manager 150 may determine which regions 255 the new feature vectors 405 fall under. Based on the identified regions 255, the schedule manager 150 may determine the behavioral pattern of the user 302. For example, if the feature vectors 265 fall into the region 255 associated with users that interact between noon and 3:00 pm on Mondays, the schedule manager 150 may classify the user 302 as such.

Based on the behavioral pattern, the schedule manager 150 may select or identify the category 270 for the user 302. The category 270 may correspond to the group (or cohort) of users with similar behavioral patterns as the user 302 based on the event dataset 305. The schedule manager 150 may identify or select the new schedule 410 corresponding to the category 270. In some embodiments, the schedule manager 150 may select the new schedule 410 pre-generated for the category 270. For example, the schedule manager 150 may identify the new schedule 410 generated from another user in the category 270. In some embodiments, schedule manager 150 may generate the new schedule 410 using the one or more regions 255 associated with the category 270. For each region 255 associated with the category 270, the schedule manager 150 may determine or identify the centroid 260 defining the region 255. Based on the centroid 260, the schedule manager 150 may calculate or determine a corresponding delivery time 430 to insert the new schedule 410. In some embodiments, using the centroid 260, the schedule manager 150 may also select or identify the corresponding message 435 for the delivery time 430.

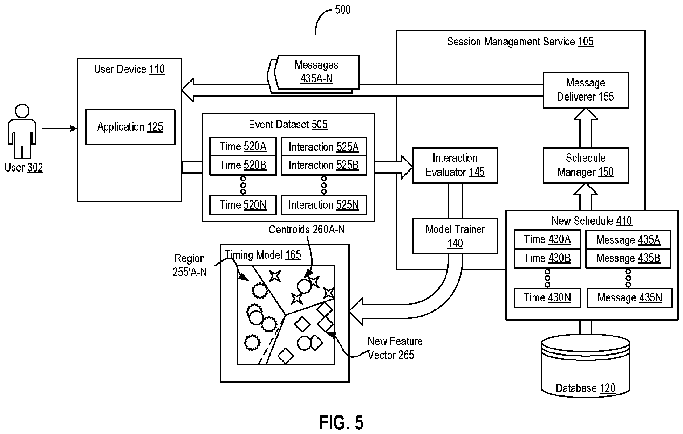

Referring now to FIG. 5, depicted is a block diagram of a process 500 of providing a message 435 according to the new schedule 410 to obtain a subsequent event dataset 505' to update the timing model 165. The process 500 may include or correspond to operations performed by the system 100 to provide the messages 435 and update the timing model 165. Under the process 500, the message deliverer 155 may output, transmit, or otherwise provide at least one message 435 at the delivery time 430 for presentation on the user device 110 in accordance with the new schedule 410. When the schedule manager 150 extracts the delivery time 430 to send the one or more messages 435, the message deliverer 155 may create, generate, or use a data structure to store the messages 435 until the associated delivery time 430 occurs. The message deliverer 155 may store the messages 435 in, for example, a queue, a stack, a linked list, an array, a hash map, a heap, a multiset, among others, until the delivery time 430 occurs. For instance, the message deliverer 155 may store the messages 435 in a linked list where the head of the linked list is the first message 435A at a delivery time 330A in the early morning and the tail of the linked list is the last message 435B at a delivery time 330B in the late night.

The message deliverer 155 may transmit, send, or otherwise provide the message to the user device 110, responsive to the delivery time 430 for the message 435 occurring. The message deliverer 155 may transmit the set of messages 435 to the user device 110 at the same delivery time 430. In some embodiments, the message deliverer 155 may transmit the set of messages 435 at different delivery times 430. Each message 435 may correspond to at least one delivery time 430 within the schedule. For instance, according to the new schedule 410, the message deliverer 155 may transmit a first message 435A, a second message 435B, and a third message 435C at different delivery times 430A-C in the morning at 6:15 am, 7:00 am, and 8:30 am to the user device 110. The message deliverer 155 may transmit a fourth message 435D and a fifth message 435E over a short span of times 330D and 330E in the late afternoon at 3:45 pm and 3:50 pm to the user device 110 to be presented as chat bot messages (e.g., displayed within a chat box message interface of the application 125).

Upon receipt, the user device 110 may present, provide, or otherwise display the messages 435. In some embodiments, the application 125 on the user device 110 may present the message 335 using the UI elements of the user device 110. The messages 435 may correspond to treatments, therapeutics, exercises, therapy, and medications, among others, for the user 302 to do or execute. For instance, the message 435 may encourage the user 302 to conduct breathing exercises to treat asthma. In another instance, the messages 335 may encourage the user 302 to ingest tablets for pain associated with lung cancer. In another instance, the message 435 may encourage the user 302 to perform physical therapy exercises for leg surgery recovery. By presenting the messages 435 at the delivery time 430, the user device 110 may capture more interactions between the user 302 and the application 125. The user device 110 (or the application 125) may monitor for interactions by the user 302 with the user device 110, in response to the provision of the message 435.

As the user device 110 detects, captures, and otherwise identifies interactions, the user device 110 may generate an event dataset 505. The event dataset 505 may be generated in a similar manner as the generation of the event dataset 305. In some embodiments, the application 125 may generate the event dataset 505 for an interval (e.g., every 1 day to 4 weeks) within the time window (e.g., 5 days to 1 month) for the initiation schedule 510. In some embodiments, the application 125 may generate the event dataset 505 at or towards (e.g., within 1 to 5 days) an end of the time window for the initiation schedule 510.

The event dataset 505 may include a set of interaction times 520A-N (hereinafter generally referred to as interaction times 520) and a corresponding set of interaction 525A-N (hereinafter generally referred to as interactions 525). Each interaction time 520 may identify a time of day, a day of week, or a date (e.g., year, month, and day), among others for the associated interaction 525. In some embodiments, the interaction time 520 may be relative to another set reference time, such as a start of therapy or a start of week, among others. Continuing on, the interaction 525 may identify a type of interaction (e.g., mouse click, a swipe, a hover over, tilt, or a screen touch), a type of activity (e.g., performed by the user 502 and recorded via the application 125), an indication of whether the activity was performed, among others. The user device 110 may generate the event dataset 505 by using the application 125 to collect, store, or otherwise capture each interaction 525 and the respective interaction time 520. Each interaction time 520 may be a length of time or an instant of time. For instance, an interaction time 520A may correspond to a plurality of interactions 525 occurring within a time window of 2-6 minutes. In another instance, an interaction time 520Amay correspond to a single interaction 525A.

The user device 110 may transmit, send, or otherwise provide the event dataset 305 to the interaction evaluator 145. The user device 110 may transmit a sending request to the session management service 105. The sending request may indicate that the user device 110 is online and has an event dataset 505 to send. The sending request may include metadata associated with the event dataset 505 such as the size of the event dataset 505, the duration in which the user device 110 was offline, the user device 110 corresponding to the event dataset 505, the device ID, among others. Upon connecting, the user device 110 may transmit the event dataset 505 to the session management service 105.

The interaction evaluator 145 may obtain, receive, or otherwise retrieve the event dataset 505 from the user device 110. Upon receipt, the interaction evaluator 145 may parse the event dataset 505 to extract or identify the set of interaction times 520 and the corresponding set of interactions 525. To evaluate the initiation schedule 510, the interaction evaluator 145 may compare the delivery time 530 with the initiation schedule 510 with the interaction time 520 within the event dataset 505 and output a performance score. The performance score may indicate a level of effectiveness for the set of delivery times 530 of the initiation schedule 510 in accordance with the number of interactions 525. In some instances, the interaction time 520 within the event dataset 505 may not deviate from the time within the initiation schedule 510 indicating a performance score of high effectiveness of the initiation schedule 510. In some instances, the interaction time 520 within the event dataset 505 may deviate from the time within the initiation schedule 510 indicating a performance score of low effectiveness of the initiation schedule 510. The interaction evaluator 145 may convey, send, or otherwise provide the event dataset 505 and the new schedule 410 to the model trainer 140.

In some embodiments, the interaction evaluator 145 may transmit, send, or otherwise provide the performance score to the model trainer 140. The performance score may be used to update the timing model 165. For instance, a performance score of high effectiveness for the new schedule 410, may improve the timing model 165 by including parameters to fine tune the timing model 165. Conversely, a performance score of low effectiveness may generate new feature vectors 405 with associations in accordance with the interaction time 520 of the event dataset 505 to improve the regions 255 of the timing model 165. In some embodiments, the interaction evaluator 145 may provide the event dataset 505 and the associated new schedule 410, when the performance score satisfies a threshold.

Using the event dataset 505 and the associated new schedule 410, the model trainer 140 may update the timing model 165. The updating of the timing model 165 may be similar to the initiation, training, and establishment of the timing model 165 as detailed herein. For instance, the model trainer 140 may create new feature vector 405 within a respective region 255' A-N (hereinafter generally referred to as region 255') to update the timing model 165. In some embodiments, the model trainer 140 may continuously train the timing model 165 until the timing model 165 reaches convergence for each user of the application 125. Using the new feature vectors 405, the model trainer 140 may populate the regions 255 with the interaction times 520 that may improve the timing model 165. In some embodiments, the model trainer 140 may create another instance of the timing model 165 for the user 302. The timing model 165 may be particular to the user 302 to generate schedules. With the creation, the model trainer 140 may train the new instance of the timing model 165 using the event dataset 505 (and previous event datasets for the user 302) to modify or update the regions 255 defined in the feature space 250. For example, the model trainer 140 may add at least one new feature vector 265' to update the regions 255' of the features space 250 of the timing model 165. The timing model 165 may update through multiple iterations of generating a schedule 410 and receiving the event dataset 505.

In this manner, using the systems and methods described herein, the session management service 105 may provide messages 435 at the optimal times at which the user 302 is most likely to respond. Furthermore, the session management service 105 may increase adherence over the course of digital therapeutic treatment delivered via the application 125. Pairing the model trainer 140 with the interaction evaluator 145, may allow the session management service 105 to aggregate real-time data associated with the user 302 over a specified time period. The interaction evaluator 145 may provide the model trainer 140 with the behavioral pattern changes to establish real-time adjustments of the timing model 165, all the while limiting the number of invocations with data aggregated over specified time periods. The model trainer 140 may further update the timing model 165 to adapt to changes in the behavioral pattern of the user 302. The schedule manager 150 may generate and select the initiation schedule 310 and new schedule 410 for the respective user 302, by using the timing model 165. By providing messages at the optimal times in accordance with the selected schedule (initiation schedule 310 or the new schedule 410), the session management service 105 may reduce resource consumption of computing resources and network bandwidth that would have otherwise been spent in transmitting messages that are less effective.

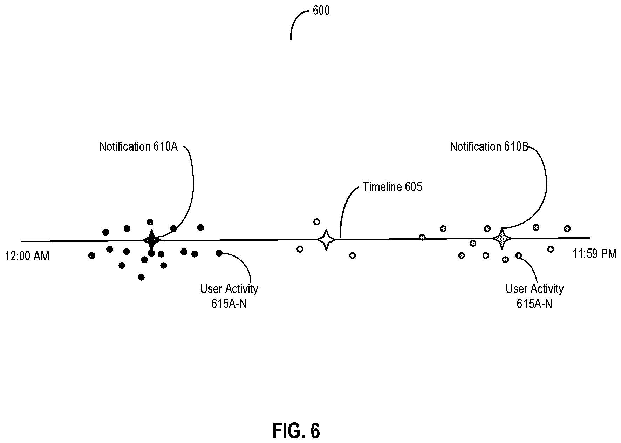

Referring now to FIG. 6, depicted is an example of a feature space 600 for the event dataset (i.e., event dataset 305, event dataset 505). The feature space 600 may include a timeline 605 identifying a set of data points corresponding to notifications 610A and 610B and a set of data points corresponding to user activity 615A-N. Throughout a given day different users may interact with their respective instances of the application 125 at different times relative to other users. For instance, the notification 610A may occur in the morning which allows for a high amount of user activity 615A-N in the morning. In contrast, the notification 610B may be sent in the evening which may allow for high user activity 615A-N in the evening. The high activity may occur based on behavioral patterns such as exercise routines, sleep schedule, work hours, dietary habits, among others.

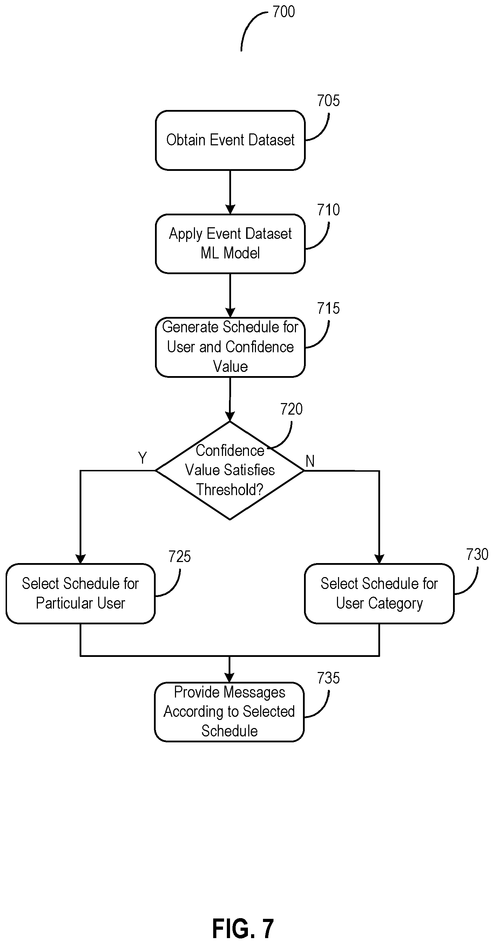

Referring now to FIG. 7, depicted is a flow diagram of a method 700 of adaptive generation of desired times to deliver messages through a digital therapeutics' application. The method 700 may be implemented or performed using any of the components described herein, such as the session management service 105 and the user device 110, or any combination thereof. Under the method 700, a computing system (e.g., the session management service 105 or the user device 110 may obtain an event dataset (705). The computing system may apply an event dataset to a ML model (710). The computing system may generate a schedule for the user and an associated confidence value (715). The computing system may determine whether the confidence value satisfies a threshold. The computing system may select a schedule for a particular user (720). The computing system may select a schedule for a user category (725). The computing system may provide messages according to the schedule (730).

### B. Network and Computing Environment

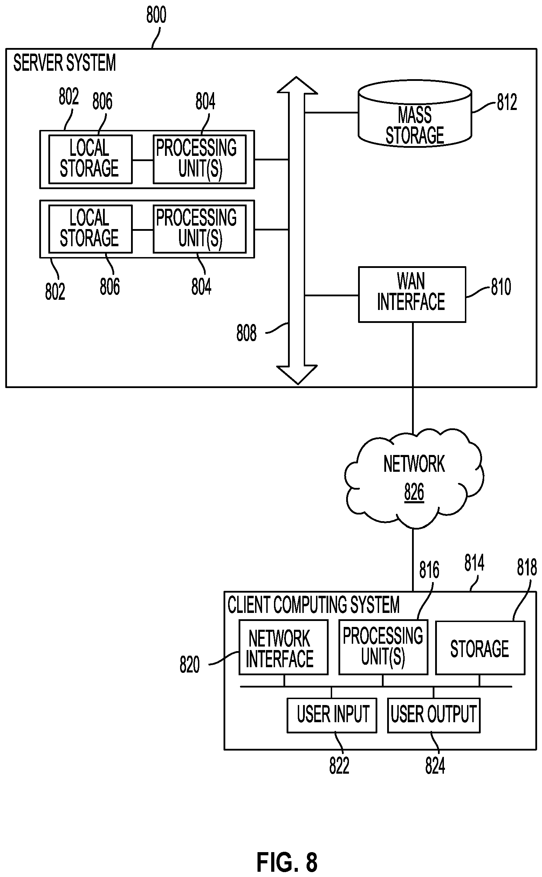

Various operations described herein can be implemented on computer systems. FIG. 8 shows a simplified block diagram of a representative server system 800, client computer system 814, and network 826 usable to implement certain embodiments of the present disclosure. In various embodiments, server system 800 or similar systems can implement services or servers described herein or portions thereof. Client computer system 814 or similar systems can implement clients described herein. The system 100 described herein can be similar to the server system 800. Server system 800 can have a modular design that incorporates a number of modules 802 (e.g., blades in a blade server embodiment); while two modules 802 are shown, any number can be provided. Each module 802 can include processing unit(s) 804 and local storage 806.

Processing unit(s) 804 can include a single processor, which can have one or more cores, or multiple processors. In some embodiments, processing unit(s) 804 can include a general-purpose primary processor as well as one or more special-purpose co-processors, such as graphics processors, digital signal processors, or the like. In some embodiments, some, or all processing units 804 can be implemented using customized circuits, such as application specific integrated circuits (ASICs) or field programmable gate arrays (FPGAs). In some embodiments, such integrated circuits execute instructions that are stored on the circuit itself. In other embodiments, processing unit(s) 804 can execute instructions stored in local storage 806. Any type of processors in any combination can be included in processing unit(s) 804.

Local storage 806 can include volatile storage media (e.g., DRAM, SRAM, SDRAM, or the like) and/or non-volatile storage media (e.g., magnetic or optical disk, flash memory, or the like). Storage media incorporated in local storage 806 can be fixed, removable, or upgradeable as desired. Local storage 806 can be physically or logically divided into various subunits such as a system memory, a read-only memory (ROM), and a permanent storage device. The system memory can be a read-and-write memory device or a volatile read-and-write memory, such as dynamic random-access memory. The system memory can store some or all of the instructions and data that processing unit(s) 804 need at runtime. The ROM can store static data and instructions that are needed by processing unit(s) 804. The permanent storage device can be a non-volatile read-and-write memory device that can store instructions and data even when module 802 is powered down. The term "storage medium" as used herein includes any medium in which data can be stored indefinitely (subject to overwriting, electrical disturbance, power loss, or the like) and does not include carrier waves and transitory electronic signals propagating wirelessly or over wired connections.

In some embodiments, local storage 806 can store one or more software programs to be executed by processing unit(s) 804, such as an operating system and/or programs implementing various server functions, such as functions of the system 100 or any other system described herein, or any other server(s) associated with system 100 or any other system described herein.

"Software" refers generally to sequences of instructions that, when executed by processing unit(s) 804, cause server system 800 (or portions thereof) to perform various operations, thus defining one or more specific machine embodiments that execute and perform the operations of the software programs. The instructions can be stored as firmware residing in read-only memory and/or program code stored in non-volatile storage media that can be read into volatile working memory for execution by processing unit(s) 804. Software can be implemented as a single program or as a collection of separate programs or program modules that interact as desired. From local storage 806 (or non-local storage described below), processing unit(s) 804 can retrieve program instructions to execute, and data to process in order to execute various operations described above.

In some server systems 800, multiple modules 802 can be interconnected via a bus or other interconnect 808, forming a local area network that supports communication between modules 802 and other components of server system 800. Interconnect 808 can be implemented using various technologies, including server racks, hubs, routers, etc.

A wide area network (WAN) interface 810 can provide data communication capability between the local area network (e.g., through the interconnect 808) and the network 826, such as the Internet. Other technologies can be used to communicatively couple the server system with the network 826, including wired (e.g., Ethernet, IEEE 802.3 standards) and/or wireless technologies (e.g., Wi-Fi, IEEE 802.11 standards).

In some embodiments, local storage 806 is intended to provide working memory for processing unit(s) 804, providing fast access to programs and/or data to be processed while reducing traffic on interconnect 808. Storage for larger quantities of data can be provided on the local area network by one or more mass storage subsystems 812 that can be connected to interconnect 808. Mass storage subsystem 812 can be based on magnetic, optical, semiconductor, or other data storage media. Direct attached storage, storage area networks, network-attached storage, and the like can be used. Any data stores or other collections of data described herein as being produced, consumed, or maintained by a service or server can be stored in mass storage subsystem 812. In some embodiments, additional data storage resources may be accessible via WAN interface 810 (potentially with increased latency).

Server system 800 can operate in response to requests received via WAN interface 810. For example, one of modules 802 can implement a supervisory function and assign discrete tasks to other modules 802 in response to received requests. Work allocation techniques can be used. As requests are processed, results can be returned to the requester via WAN interface 810. Such operation can generally be automated. Further, in some embodiments, WAN interface 810 can connect multiple server systems 800 to each other, providing scalable systems capable of managing high volumes of activity. Other techniques for managing server systems and server farms (collections of server systems that cooperate) can be used, including dynamic resource allocation and reallocation.

Server system 800 can interact with various user-owned or user-operated devices via a wide-area network such as the Internet. An example of a user-operated device is shown in FIG. 8 as client computing system 814. Client computing system 814 can be implemented, for example, as a consumer device such as a smartphone, other mobile phone, tablet computer, wearable computing device (e.g., smart watch, eyeglasses), desktop computer, laptop computer, and so on.

For example, client computing system 814 can communicate via WAN interface 810. Client computing system 814 can include computer components such as processing unit(s) 816, storage device 818, network interface 820, user input device 822, and user output device 824. Client computing system 814 can be a computing device implemented in a variety of form factors, such as a desktop computer, laptop computer, tablet computer, smartphone, other mobile computing device, wearable computing device, or the like.

Processing unit 816 and storage device 818 can be similar to processing unit(s) 804 and local storage 806 described above. Suitable devices can be selected based on the demands to be placed on client computing system 814. For example, client computing system 814 can be implemented as a "thin" client with limited processing capability or as a high-powered computing device. Client computing system 814 can be provisioned with program code executable by processing unit(s) 816 to enable various interactions with server system 800.

Network interface 820 can provide a connection to the network 826, such as a wide area network (e.g., the Internet) to which WAN interface 810 of server system 800 is also connected. In various embodiments, network interface 820 can include a wired interface (e.g., Ethernet) and/or a wireless interface implementing various RF data communication standards such as Wi-Fi, Bluetooth, or cellular data network standards (e.g., 3G, 4G, LTE, etc.).

User input device 822 can include any device (or devices) via which a user can provide signals to client computing system 814; client computing system 814 can interpret the signals as indicative of particular user requests or information. In various embodiments, user input device 822 can include any or all of a keyboard, touch pad, touch screen, mouse, or other pointing device, scroll wheel, click wheel, dial, button, switch, keypad, microphone, and so on.

User output device 824 can include any device via which client computing system 814 can provide information to a user. For example, user output device 824 can include display-to-display images generated by or delivered to client computing system 814. The display can incorporate various image generation technologies, e.g., a liquid crystal display (LCD), a light-emitting diode (LED) display including organic light-emitting diodes (OLED), a projection system, a cathode ray tube (CRT), or the like, together with supporting electronics (e.g., digital-to-analog or analog-to-digital converters, signal processors, or the like). Some embodiments can include a device such as a touchscreen that functions as both an input and output device. In some embodiments, other user output devices 824 can be provided in addition to or instead of a display. Examples include indicator lights, speakers, tactile "display" devices, printers, and so on.

Some embodiments include electronic components, such as microprocessors, storage, and memory that store computer program instructions in a computer readable storage medium. Many of the features described in this specification can be implemented as processes that are specified as a set of program instructions encoded on a computer readable storage medium. When one or more processing units execute these program instructions, they cause the processing unit(s) to perform various operations indicated in the program instructions. Examples of program instructions or computer code include machine code, such as is produced by a compiler, and files including higher-level code that are executed by a computer, an electronic component, or a microprocessor using an interpreter. Through suitable programming, processing unit(s) 804 and 816 can provide various functionality for server system 800 and client computing system 814, including any of the functionality described herein as being performed by a server or client, or other functionality.

It will be appreciated that server system 800 and client computing system 814 are illustrative and that variations and modifications are possible. Computer systems used in connection with embodiments of the present disclosure can have other capabilities not specifically described here. Further, while server system 800 and client computing system 814 are described with reference to particular blocks, it is to be understood that these blocks are defined for convenience of description and are not intended to imply a particular physical arrangement of component parts. For instance, different blocks can be, but need not be located in the same facility, in the same server rack, or on the same motherboard. Further, the blocks need not correspond to physically distinct components. Blocks can be configured to perform various operations, e.g., by programming a processor or providing appropriate control circuitry, and various blocks might or might not be reconfigurable depending on how the initial configuration is obtained. Embodiments of the present disclosure can be realized in a variety of apparatus including electronic devices implemented using any combination of circuitry and software.

While the disclosure has been described with respect to specific embodiments, one skilled in the art will recognize that numerous modifications are possible. Embodiments of the disclosure can be realized using a variety of computer systems and communication technologies, including, but not limited to, specific examples described herein. Embodiments of the present disclosure can be realized using any combination of dedicated components and/or programmable processors and/or other programmable devices. The various processes described herein can be implemented on the same processor or different processors in any combination. Where components are described as being configured to perform certain operations, such configuration can be accomplished, e.g., by designing electronic circuits to perform the operation, by programming programmable electronic circuits (such as microprocessors) to perform the operation, or any combination thereof. Further, while the embodiments described above may make reference to specific hardware and software components, those skilled in the art will appreciate that different combinations of hardware and/or software components may also be used and that particular operations described as being implemented in hardware might also be implemented in software or vice versa.

Computer programs incorporating various features of the present disclosure may be encoded and stored on various computer readable storage media; suitable media include magnetic disk or tape, optical storage media such as compact disk (CD) or digital versatile disk (DVD), flash memory, and other non-transitory media. Computer readable media encoded with the program code may be packaged with a compatible electronic device, or the program code may be provided separately from electronic devices (e.g., via Internet download or as a separately packaged computer-readable storage medium).

Thus, although the disclosure has been described with respect to specific embodiments, it will be appreciated that the disclosure is intended to cover all modifications and equivalents within the scope of the following claims.

## Claims

1. A method of generating times for providing messages over networked environments, comprising:
obtaining, by one or more processors, for a user device, an event dataset identifying a plurality of interaction times corresponding to a plurality of interactions over an instant time window by a user with an application on the user device to address a condition of the user;
applying, by the one or more processors, the event dataset to a machine learning (ML) model, wherein the ML model is established using a training dataset including a plurality of examples, each of the plurality of examples including a respective event dataset identifying a respective plurality of interaction times corresponding to a respective plurality of interactions over a respective time window by a respective user with a respective application on a respective user device, at least one of the respective plurality of interaction detected in response to provision of one or more messages;
generating, by the one or more processors, based on applying the event dataset to the ML model, a defined time at which a message is to be provided to the user device during a subsequent time window; and
providing, by the one or more processors, for presentation on the user device to address the condition of the user, the message in accordance with the defined time.

2. The method of claim 1, wherein the ML model comprises a clustering model defining a plurality of clusters established using the plurality of examples, each cluster of the plurality of clusters corresponding to a respective subset of the plurality of interaction times, each cluster of the plurality of clusters associated with a respective defined time at which to provide a respective message of the plurality of messages within the set time window

3. The method of claim 2, wherein generating the defined time further comprises:
comparing the plurality of interaction times of the event dataset for the user with one or more of the plurality of clusters of the clustering model,
identifying, from the plurality of clusters, a cluster which corresponds to at least one of the plurality of interaction times, and
using the respective defined time of the cluster as the defined time.

4. The method of claim 2, wherein generating the defined time further comprises:
comparing the plurality of interaction times of the event dataset for the user with one or more of the plurality of clusters of the clustering model,
identifying, from the plurality of clusters, a subset of clusters for a corresponding subset of the plurality of interaction times, and
using the respective defined times of each of the subset of clusters as a plurality of defined times for a schedule.

5. The method of claim 1, wherein generating the defined time further comprises generating a schedule identifying, for each defined time of the plurality of defined times, a respective message type for a corresponding message of the plurality of messages to be provided to the user device.

6. The method of claim 1, wherein at least one of the plurality of examples of the training dataset further identifies at least one of: (i) a respective schedule identifying a corresponding plurality of defined times at which a respective plurality of messages is to be provided to the respective user device over the set time window, or (ii) a respective indication of whether the respective schedule increased a corresponding response rate of respective plurality of interactions by at least a threshold.

7. The method of claim 1, wherein generating the defined time further comprises generating, based on applying the event dataset to the ML model, a score indicating a degree of confidence for the defined time, and
determining, by the one or more processors, that the defined time is to be used to provide the message to the user device, responsive to the score satisfying a threshold.

8. The method of claim 1, further comprising:
receiving, by the one or more processors, a subsequent event dataset identifying a subsequent plurality of interaction times corresponding to a subsequent plurality of interactions over the subsequent time window by the user with the application in response to provision of at least one of the plurality of messages; and
updating, by the one or more processors, the ML model using the subsequent event data and the defined time.

9. The method of claim 1, further comprising:
selecting, by the one or more processors, prior to obtaining the event dataset, an initiation schedule identifying an initial plurality of defined times at which an initial plurality of messages is to be provided to the user device over the initial time window, responsive to identifying a lack of prior event datasets for the user; and
providing, by the one or more processors, for presentation on the user device, the initial plurality of messages in accordance with the initial plurality of defined times of the initiation schedule, and
wherein obtaining the event dataset further comprises obtaining the event dataset identifying the plurality of interaction times corresponding to the plurality of interactions by the user with the application in response to presentation of at least one of the initial plurality of messages of the initiation schedule.

10. The method of claim 1, further comprising:
determining, by the one or more processors, from a plurality of categories, a category for the user based on one or more event datasets, each category of the plurality of categories associated with a respective behavioral pattern; and
identifying, by the one or more processors, an initiation schedule based on the category determined for the user, the initiation schedule identifying an initial plurality of defined times at which an initial plurality of messages is to be provided to the user device.

11. The method of claim 1, wherein the event dataset further comprises at least one of: (i) a health metric associated with the condition of the user, (ii) an interaction rate for the plurality of interactions over the instant time window, or (iii) trait information associated with the user, at least one of the plurality of interactions corresponding to an interaction with the application independent of provision of any message.

12. The method of claim 1, wherein the message comprises at least one of a short message service (SMS) message, a multimedia messaging service (MMS), an in-app message, or a chat bot message, wherein the message is to be presented to the user, at least in partial concurrence with the user being on a medication to address the condition.

13. A system for generating times for providing messages over networked environments, comprising one or more processors configured to perform the method of any one preceding claim.

14. A computer-readable storage medium comprising computer-readable instructions that, when executed by one or more processors, cause the one or more processors to perform the method of any one of claims 1 to 12.

## Amended claims

### Amended claims in accordance with Rule 137(2) EPC.

1. A method of generating times for providing content over networked environments, comprising:
obtaining, by one or more processors, for a user device, an event dataset identifying a plurality of interaction times corresponding to a plurality of interactions over an instant time window by a user with an application on the user device to address a condition of the user;
applying, by the one or more processors, the event dataset to a machine learning, ML, model, wherein the ML model is established using a training dataset including a plurality of examples, each of the plurality of examples including a respective event dataset identifying a respective plurality of interaction times corresponding to a respective plurality of interactions over a respective time window by a respective user with a respective application on a respective user device, at least one of the respective plurality of interaction detected in response to provision of content;
generating, by the one or more processors, based on applying the event dataset to the ML model, a delivery time based at least on a plurality of interactions identified in the event dataset, the delivery time corresponding to a likelihood of a future user interaction; and
providing, by the one or more processors, instructions to cause presentation of content on the user device at a time corresponding with the delivery time.

2. The method of claim 1, wherein the ML model comprises a clustering model defining a plurality of clusters established using the plurality of examples, each cluster of the plurality of clusters corresponding to a respective subset of the plurality of interaction times, each cluster of the plurality of clusters associated with a respective delivery time at which to provide respective content within a set time window.

3. The method of claim 2, wherein generating the delivery time further comprises:
comparing the plurality of interaction times of the event dataset for the user with one or more of the plurality of clusters of the clustering model,
identifying, from the plurality of clusters, a cluster which corresponds to at least one of the plurality of interaction times, and
using the respective delivery time of the cluster as the delivery time.

4. The method of claim 2, wherein generating the delivery time further comprises:
comparing the plurality of interaction times of the event dataset for the user with one or more of the plurality of clusters of the clustering model,
identifying, from the plurality of clusters, a subset of clusters for a corresponding subset of the plurality of interaction times, and
using the respective delivery times of each of the subset of clusters as a plurality of delivery times for a schedule.

5. The method of claim 1, wherein generating the delivery time further comprises generating a schedule identifying, for each delivery time of a plurality of delivery times, a respective content type for corresponding content to be provided to the user device.

6. The method of claim 1, wherein at least one of the plurality of examples of the training dataset further identifies at least one of: (i) a respective schedule identifying a corresponding plurality of delivery times at which respective content is to be provided to the respective user device over a set time window, or (ii) a respective indication of whether the respective schedule increased a corresponding response rate of respective plurality of interactions by at least a threshold.

7. The method of claim 1, wherein generating the delivery time further comprises generating, based on applying the event dataset to the ML model, a score indicating a degree of confidence for the delivery time, and
determining, by the one or more processors, that the delivery time is to be used to provide the content to the user device, responsive to the score satisfying a threshold.

8. The method of claim 1, further comprising:
receiving, by the one or more processors, a subsequent event dataset identifying a subsequent plurality of interaction times corresponding to a subsequent plurality of interactions over the subsequent time window by the user with the application in response to provision of the content; and
updating, by the one or more processors, the ML model using the subsequent event data and the delivery time.

9. The method of claim 1, further comprising:
selecting, by the one or more processors, prior to obtaining the event dataset, an initiation schedule identifying an initial plurality of delivery times at which an initial plurality of content is to be provided to the user device over an initial time window, responsive to identifying a lack of prior event datasets for the user; and
providing, by the one or more processors, for presentation on the user device, the initial content in accordance with the initial plurality of delivery times of the initiation schedule,
wherein obtaining the event dataset further comprises obtaining the event dataset identifying the plurality of interaction times corresponding to the plurality of interactions by the user with the application in response to presentation of at least one of the content of the initiation schedule.

10. The method of claim 1, further comprising:
determining, by the one or more processors, from a plurality of categories, a category for the user based on one or more event datasets, each category of the plurality of categories associated with a respective behavioral pattern; and
identifying, by the one or more processors, an initiation schedule based on the category determined for the user, the initiation schedule identifying an initial plurality of delivery times at which an initial plurality of messages is to be provided to the user device.

11. The method of claim 1, wherein the event dataset further comprises at least one of: (i) a health metric associated with the condition of the user, (ii) an interaction rate for the plurality of interactions, or (iii) trait information associated with the user, at least one of the plurality of interactions corresponding to an interaction with the application independent of provision of any content.

12. The method of claim 1, wherein the content comprises at least one of a short message service, SMS, message, a multimedia messaging service, MMS, an in-app message, or a chat bot message, wherein the message is to be presented to the user, at least in partial concurrence with the user being on a medication to address the condition.

13. A system for generating times for providing content over networked environments, comprising one or more processors configured to perform the method of any one preceding claim.

14. A computer-readable storage medium comprising computer-readable instructions that, when executed by one or more processors, cause the one or more processors to perform the method of any one of claims 1 to 12.
